# EUROPÄISCHE PATENTANMELDUNG

(11) **EP 3 305 752 A1**
(43) Veröffentlichungstag der Anmeldung: **11.04.2018**
(21) Anmeldenummer: 16192357.8
(22) Anmeldetag: 05.10.2016
(51) Int. Cl.: C07C 37/84, C07C 39/15

(54) **VERFAHREN ZUR ISOLIERUNG VON BIPHENOLEN UNTER EINSATZ VON ESSIGSÄURE UND EIS**

(71) Anmelder: Evonik Degussa GmbH, 45128 Essen (DE)
(72) Erfinder: WALDVOGEL, Siegfried R., 55435 Gau-Algesheim (DE); QUELL, Thomas, 45657 Recklinghausen (DE); SELT, Maximillan, 55118 Mainz (DE); FRANKE, Robert, 45772 Marl (DE); DYBALLA, Katrin Marie, 45657 Recklinghausen (DE); FRIDAG, Dirk, 45721 Haltern am See (DE)

(57) **Zusammenfassung**

Verfahren zur Isolierung von Biphenolen unter Einsatz von Essigsäure und Eis.

## Beschreibung

Die Erfindung betrifft ein Verfahren zur Isolierung von Biphenolen unter Einsatz von Essigsäure und Eis.

Biphenole sind wichtige Ligand bzw. Ligandenbaustein in vielen großtechnisch angewendeten Katalysen. Um wirtschaftlich nutzbare Liganden herzustellen, muss der Zugang zu Biphenolen so effizient wie möglich sein.

Methoden, welche die Herstellung von Biphenolen ermöglichen, verwenden vorwiegen säulenchromatographische Trennverfahren. Solchen Techniken führen zu einem hohen Verbrauch an Kieselgel und Lösungsmitteln. (Q. Jiang, W. Sheng, M. Tian, J. Tang, C. Guo, Eur. J. Org. Chem. 2013, 2013, 1861-1866).

Die bisher bekannten Lösungen zur Isolierung von Biphenolen sind sehr zeit- und energieintensiv: Säulenchromatographische Trennverfahren benötigen sowohl eine stationäre Phase (meist Kieselgel), die nicht vollständig wieder verwendbar ist, als auch eine mobile Phase. Die Lösungsmittel, welche als mobile Phase genutzt werden, können zwar zum Teil destillativ zurückgewonnen werden, allerdings ist dafür ein hohes Maß an Energie notwendig. Die Menge an benötigtem Lösungsmittel macht das Verfahren zusätzlich teuer und ineffizient. Die Aufarbeitung mittels Sublimation erfordert einen hohen apparativen Aufwand, um das notwendige Vakuum zu erzeugen. Dabei ist besonders die Skalierung in den technischen Maßstab schwierig zu realisieren. Abgesehen von den technischen Schwierigkeiten ist bei der Sublimation auch noch der Energieaufwand sehr groß.

Der Erfindung lag die Aufgabe zugrunde, ein Verfahren bereitzustellen, welches die in Zusammenhang mit dem Stand der Technik beschriebenen Nachteile nicht aufweist. Insbesondere so der apparative Aufwand klein sein und nur eine vergleichsweise geringe Menge an Lösungsmittel benötigt werden. Das Verfahren sollt möglichst auf Techniken basieren, die auch im industriellen Maßstab umgesetzt werden können.

Gelöst wird die Aufgabe durch eine Verfahren nach Anspruch 1 oder 2.

Verfahren umfassend die Verfahrensschritte:
a1) bereitstellen einer Verbindung gemäß der Formel (I): 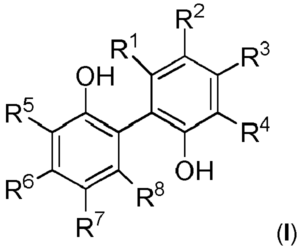 wobei die Reste R¹, R², R³, R⁴, R⁵, R⁶, R¹, R⁸ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen;
   im Form eines festen Rohproduktes;
b1) lösen des Rohproduktes in Essigsäure;
c1) gießen der in b1) erhaltenen Lösung auf Eis oder zugeben von Eis zu der in b1) erhaltenen Lösung, so dass die Verbindung gemäß der Formel (I) als Feststoff ausfällt.

Unter "Rohprodukt" wird in Zusammenhang mit dieser Erfindung ein bei der Synthese anfallendes Produkt verstanden, welches noch Verunreinigungen aufweist. Mögliche Verunreinigungen sind hierbei beispielsweise: die bei der Synthese anfallenden Nebenprodukte, nicht umgesetzte Ausgangstoffe, zugesetzte Hilfsstoffe, Katalysatoren oder Lösungsmittel.

Dieses erste Verfahren geht von einem als Feststoff isoliertem Rohprodukt aus.

Alternativ kann auch die Reaktionslösung aus der Synthese des Biphenols direkt verwendet werden, ohne dass das Biphenol als festes Rohprodukt, oder in irgendeiner anderen Form aus der Reaktionslösung isoliert wird.

Verfahren umfassend die Verfahrensschritte:
a2) bereitstellen einer Lösung aus einem Reaktionsgemisch, welches nach der Synthese der Verbindung (I) vorliegt, 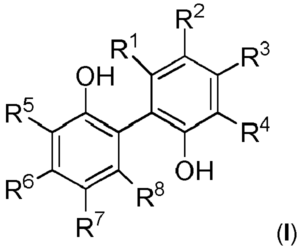 wobei die Reste R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen; wobei die Lösung umfasst:
   - ein Lösungsmittel,
   - die Verbindung (I),
   - Verunreinigungen, welche bei der Synthese angefallen sind;
b2) für den Fall, dass es sich bei dem Lösungsmittel von a2) nicht um Essigsäure handelt, Zugabe von Essigsäure;
c2) gießen der in b2) erhaltenen Lösung auf Eis oder zugeben von Eis zu der in b2) erhaltenen Lösung, so dass die Verbindung gemäß der Formel (I) als Feststoff ausfällt.

Mögliche Verunreinigungen sind hierbei beispielsweise: die bei der Synthese anfallenden Nebenprodukte, nicht umgesetzte Ausgangstoffe, zugesetzte Hilfsstoffe, Katalysatoren oder weitere Lösungsmittel.

Die im Folgenden beschrieben Variationen beziehen sich jeweils auf beide der zuvor genannten Verfahren.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl.

Der Ausdruck -(C₆-C₂₀)-Aryl umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Die Erläuterungen zum Ausdruck -(C₆-C₂₀)-Aryl gelten auch für die Alkylgruppen in -O-(C₆-C₂₀)-Aryl.

Unter Halogen werden Cl, F, Br, I zusammengefasst. Hierbei ist Cl bevorzugt.

In einer Variante des Verfahrens umfasst dieses den zusätzlichen Verfahrensschritt:
d) abfiltrieren des ausgefallenen Feststoffes.

In einer Variante des Verfahrens umfasst dieses den zusätzlichen Verfahrensschritt:
e) lösen des Feststoffes aus d) in Ethylacetat und waschen der Ethylacetat-Lösung mit Wasser. Bei Bedarf kann die Ethylacetat-Lösung vor dem Waschen mit Wasser erneut filtriert werden.

In einer Variante des Verfahrens umfasst dieses den zusätzlichen Verfahrensschritt:
f) trocknen der Lösung aus e).
Das Trocken erfolgt vorzugsweise mit Magnesiumsulfat.

In einer Variante des Verfahrens umfasst dieses den zusätzlichen Verfahrensschritt:
g) abdestillieren des Lösungsmittel aus der in f) erhaltenen Lösung.

In einer Variante des Verfahrens umfasst dieses den zusätzlichen Verfahrensschritt:
h) auflösen des in g) erhaltenen Rückstandes in Cyclohexan.

In einer Variante des Verfahrens umfasst dieses den zusätzlichen Verfahrensschritt:
i) abkühlen der Lösung aus h) auf eine Temperatur in dem Bereich von 15 °C bis 7 °C, so dass ein kristalliner Feststoff ausfällt.
Vorzugsweise auf eine Temperatur in dem Bereich von 10 °C bis 7 °C.

In einer Variante des Verfahrens umfasst dieses den zusätzlichen Verfahrensschritt:
j) waschen des kristallinen Feststoffes aus i) mit Cyclohexan, wobei das Cyclohexan eine Temperatur aufweist in dem Bereich von 15 °C bis 7 °C.
Vorzugsweise eine Temperatur in dem Bereich von 10 °C bis 7 °C.

In einer Variante des Verfahrens sind die Reste R¹, R², R³, R⁴, R⁵, R⁶, R⁷, R⁸ jeweils unabhängig voneinander ausgewählt aus:

-H, -(C₁-C₁₂)-Alkyl, -O-(C₁-C₁₂)-Alkyl, -(C₆-C₂₀)-Aryl, -O-(C₆-C₂₀)-Aryl.

In einer Variante des Verfahrens sind die Reste R¹, R², R³, R⁴, R⁵, R⁶, R⁷, R⁸ jeweils unabhängig voneinander ausgewählt aus:

-H, -(C₁-C₁₂)-Alkyl, -(C₆-C₂₀)-Aryl.

In einer Variante des Verfahrens sind die Reste R¹, R², R³, R⁴, R⁵, R⁶, R⁷, R⁸ jeweils unabhängig voneinander ausgewählt aus:

-H, -(C₁-C₁₂)-Alkyl.

In einer Variante des Verfahrens stehen die Reste R¹, R³, R⁶, R⁸ für -H.

In einer Variante des Verfahrens stehen die Reste R², R⁴, R⁵, R⁷ für -(C₁-C₁₂)-Alkyl.

In einer Variante des Verfahrens stehen die Reste R², R⁴, R⁵, R⁷ für -CH₃.

In einer Variante des Verfahrens weist die Verbindung (**I**) die folgende Struktur auf:

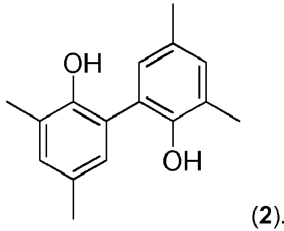

Im Folgenden wird die Erfindung anhand eines Ausführungsbeispiels näher erläutert.

Zur Realisierung der Synthese von 3,3',5,5'-Tetramethyl-2,2'-biphenol (**2**) im technischen Maßstab ist eine effiziente Aufarbeitungsmethode erforderlich, um das gewünschte Produkt von nicht umgesetztem Edukt, Nebenprodukten wie dem Pummerer Keton (**3**) oder weiteren niederwie auch hochmolekularen Verbindungen abzutrennen (Reaktionsschema 1).

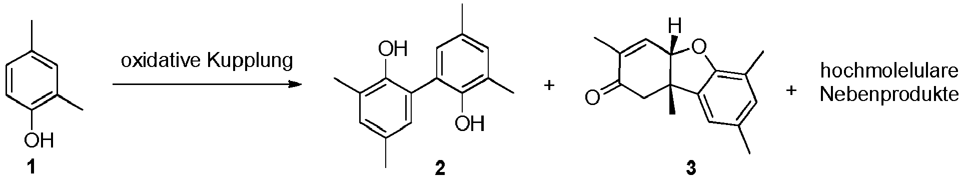

Reaktionsschema 1: Synthese von 3,3',5,5'-Tetramethyl-2,2'-biphenol (**2**) mittels oxidativer Kupplung von 2,4-Dimethylphenol (**1**).

### Analytik

### Gaschromatographie (GC/GCMS)

Die gaschromatographischen Untersuchungen (GC) von Produktgemischen und Reinsubstanzen erfolgte mit Hilfe des Gaschromatographen GC-2010 der Firma *Shimadzu,* Japan. Es wird an einer Quarzkapillarsäule ZB-5 der Firma *Phenomenex,* USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperaturfür 1 min, Heizrate: 15 °C/min, 290 °C Endtemperatur für 8 min) gemessen.

### Schmelzpunkte

Schmelzpunkte wurden mit Hilfe des Schmelzpunktbestimmungsgerätes des Typs SMP3 der Firma *Bibby Scientific Limited,* UK mit einer von Heizrate 1,0 °C/min gemessen und sind nicht korrigiert.

### Massenspektrometrie

Die Felddesorptionsmessung (FD) wurde von der massenspektrometrischen Abteilung des Institutes für Organische Chemie an einem MAT 95 der Fairma *Finnigan* gemessen.

### NMR-Spektroskopie

Die NMR-spektroskopischen Untersuchungen wurden an einem Multikernresonanz-spektrometer des Typs Avance II 400 der Firma *Bruker,* Analytische Messtechnik, Karlsruhe, durchgeführt. Als Lösungsmittel wurde CDCl3 verwendet. Die ¹H- und ¹³C-Spektren wurden gemäß dem Restgehalt an nicht deuteriertem Lösungsmittel nach der *NMR Solvent Data Chart* der Fa. *Cambridge Isotopes Laboratories,* USA, kalibriert. Die Zuordnung der ¹H- und ¹³C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Synthese und Aufreinigung

### Arbeitsvorschrift zur Synthese von 3,3',5,5'-Tetramethyl-2,2 -biphenol mittels Selendioxid

In einem Rührkessel-Reaktor mit mechanischem Rührwerk wurden 300 g 2,4-Dimethylphenol (2,5 mol) in 1800 mL Essigsäure gelöst und auf 85 °C temperiert. Nach Erreichen dieser Reaktionstemperatur wurden 190 g Selendioxid (1,7 mol, 0,7 Äquivalente) zugegeben und die Reaktion für 120 Minuten auf 85 °C gehalten. Nach Ende der Reaktion wurde die Reaktionslösung über einen Filter geleitet und auf 2340 g Eis gegeben, wobei sich ein Feststoff bildete. Dieser, fast ausschließlich aus dem gewünschten Produkt bestehende Feststoff wurde abfiltriert. Nicht umgesetztes Edukt blieb in Lösung. Der Feststoff wurde in 990 mL Ethylacetat gelöst und erneut filtriert um Reste von elementaren Selen zu entfernen. Die Ethylacetat-Lösung (= Ethylacetat-Phase) wurde drei Mal mit Wasser gewaschen, um Reste von Essigsäure zu entfernen und über Magnesiumsulfat getrocknet. Nach Destillation des Lösungsmittels wurde der Rückstand in 558 mL siedendem Cyclohexan gelöst und anschließend auf 7 °C abgekühlt (16 h). Das kristallisierte Produkt wurde abgesaugt und mit 200 mL kaltem (7 °C) Cyclohexan gewaschen. Bei nicht vollständiger Kristallisation wurde die Mutterlauge auf die Hälfte eingeengt und erneut für 16 Stunden bei 7 °C kristallisiert. Es konnten 202 g (0,8 mol) 3,3',5,5'-Tetramethyl-2,2'-biphenol mit einer Ausbeute von 68% erhalten werden.

### Charakterisierung

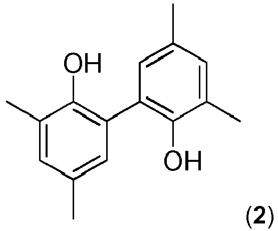

GC (Methode *hart,* ZB-5): t_{R} = 13.04 min
R_{f} (CH:EE = 9:1): 0.35
Smp.: 137 °C (aus Cyclohexan kristallisiert).
¹H-NMR (400 MHz. CDCl₃): δ[ppm] = 2.30 (s, 12H, H-7, H-8), 4.90 (s, 2H, OH), 6.88-6.89 (m, 2H, H-4), 7.01-7.02 (m, 2H, H-6).
¹³C-NMR (101 MHz, CDCl₃): δ[ppm] = 16.2 (C-7), 20.5 (C-8), 122.2 (C-1), 125.2 (C-3), 128.5 (C-6), 130.0 (C-5), 132.0 (C-4), 149.2 (C-2).
MS (FD): *m*/*z* = 242.3 [M]⁺.

Das erfindungsgemäße Verfahren basiert auf der unterschiedlichen Löslichkeit von Edukt und Produkt in einer Eis-Essigsäure-Mischung. Dadurch lässt sich das Produkt erstmals ohne aufwendige Aufarbeitungsschritte isolieren.

Nach Ende der Reaktion wird das in Essigsäure gelöste Rohprodukt oder alternativ die Lösung aus dem Reaktionsgemisch, welches nach der Synthese des Biphenols anfällt, auf Eis gegeben (oder das Eis in die Lösung), wobei das Produkt ausfällt und das Edukt in Lösung bleibt. Dadurch lässt sich das gewünschte Produkt durch einfache Filtration abtrennen. Weitere Nebenprodukte können nötigenfalls einfach durch Kristallisation aus Cyclohexan entfernt werden.

Mit dem erfindungsgemäßen Verfahren wird eine Methode bereitgestellt, welche eine Aufarbeitung ermöglicht, bei welcher der apparative Aufwand klein ist, und nur eine geringe Menge an Lösungsmittel benötigt wird, verglichen mit anderen Aufarbeitungstechniken.
Das erfindungsgemäße Verfahren kann somit gut im industriellen Maßstab umgesetzt werden. Die Aufgabe wird somit durch das erfindungsgemäße Verfahren gelöst.

## Patentansprüche

1. Verfahren umfassend die Verfahrensschritte:
a1) bereitstellen einer Verbindung gemäß der Formel (I): 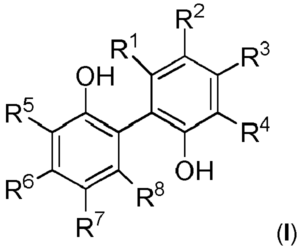 wobei die Reste R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen;
im Form eines festen Rohproduktes;
b1) lösen des Rohproduktes in Essigsäure;
c1) gießen der in b1) erhaltenen Lösung auf Eis oder zugeben von Eis zu der in b1) erhaltenen Lösung, so dass die Verbindung gemäß der Formel (I) als Feststoff ausfällt.

2. Verfahren umfassend die Verfahrensschritte:
a2) bereitstellen einer Lösung aus einem Reaktionsgemisch, welches nach der Synthese der Verbindung (I) vorliegt, 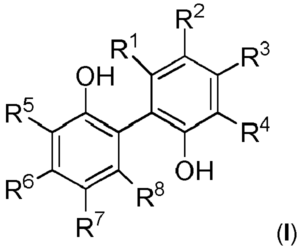
wobei die Reste R¹, R², R³, R⁴, R⁵, R⁶, R¹, R⁸ jeweils unabhängig voneinander ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl, -Halogen;
wobei die Lösung umfasst:
- ein Lösungsmittel,
- die Verbindung (**I**),
- Verunreinigungen, welche bei der Synthese angefallen sind;
b2) für den Fall, dass es sich bei dem Lösungsmittel von a2) nicht um Essigsäure handelt, Zugabe von Essigsäure;
c2) gießen der in b2) erhaltenen Lösung auf Eis oder zugeben von Eis zu der in b2) erhaltenen Lösung, so dass die Verbindung gemäß der Formel (**I**) als Feststoff ausfällt.

3. Verfahren nach einem der Ansprüche 1 oder 2,
umfassend den zusätzlichen Verfahrensschritt:
d) abfiltrieren des ausgefallenen Feststoffes.

4. Verfahren nach Anspruch 3
umfassend den zusätzlichen Verfahrensschritt:
e) lösen des Feststoffes aus d) in Ethylacetat und waschen der Ethylacetat-Lösung mit Wasser.

5. Verfahren nach Anspruch 4,
umfassend den zusätzlichen Verfahrensschritt:
f) trocknen der Lösung aus e).

6. Verfahren nach Anspruch 5,
umfassend den zusätzlichen Verfahrensschritt:
g) abdestillieren des Lösungsmittel aus der in f) erhaltenen Lösung.

7. Verfahren nach Anspruch 6,
umfassend den zusätzlichen Verfahrensschritt:
h) auflösen des in g) erhaltenen Rückstandes in Cyclohexan.

8. Verfahren nach Anspruch 7,
umfassend den zusätzlichen Verfahrensschritt:
i) abkühlen der Lösung aus h) auf eine Temperatur in dem Bereich von 15 °C bis 7 °C, so dass ein kristalliner Feststoff ausfällt.

9. Verfahren nach Anspruch 8,
umfassend den zusätzlichen Verfahrensschritt:
j) waschen des kristallinen Feststoffes aus i) mit Cyclohexan, wobei das Cyclohexan eine Temperatur aufweist in dem Bereich von 15 °C bis 7 °C.

10. Verfahren nach einem der Ansprüche 1 bis 9,
wobei die Reste R¹, R², R³, R⁴, R⁵, R⁶, R¹, R⁸ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl.

11. Verfahren nach einem der Ansprüche 1 bis 10,
wobei die Reste R¹, R², R³, R⁴, R⁵, R⁶, R⁷, R⁸ jeweils unabhängig voneinander ausgewählt sind aus:
-H, -(C₁-C₁₂)-Alkyl.

12. Verfahren nach einem der Ansprüche 1 bis 11,
wobei die Reste R¹, R³, R⁶, R⁸ für -H stehen.

13. Verfahren nach einem der Ansprüche 1 bis 12,
wobei die Reste R², R⁴, R⁵, R⁷ für -(C₁-C₁₂)-Alkyl stehen.

14. Verfahren nach einem der Ansprüche 1 bis 13,
wobei die Reste R², R⁴, R⁵, R⁷ für -CH3 stehen.

15. Verfahren nach einem der Ansprüche 1 bis 14,
wobei die Verbindung (I) die folgende Struktur aufweist:

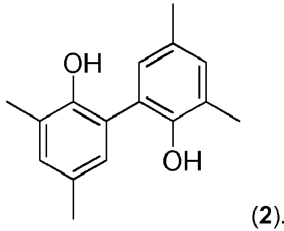

## Geänderte Patentansprüche

### Geänderte Patentansprüche gemäss Regel 137(2) EPÜ.

1. Verfahren umfassend die Verfahrensschritte:
a1) bereitstellen einer Verbindung gemäß der Formel (**2**): 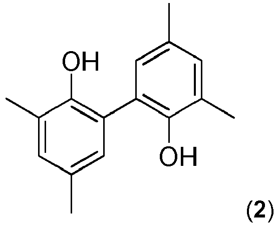 im Form eines festen Rohproduktes;
b1) lösen des Rohproduktes in Essigsäure;
c1) gießen der in b1) erhaltenen Lösung auf Eis oder zugeben von Eis zu der in b1) erhaltenen Lösung, so dass die Verbindung gemäß der Formel (**2**) als Feststoff ausfällt.

2. Verfahren umfassend die Verfahrensschritte:
a2) bereitstellen einer Lösung aus einem Reaktionsgemisch, welches nach der Synthese der Verbindung (**2**) vorliegt, 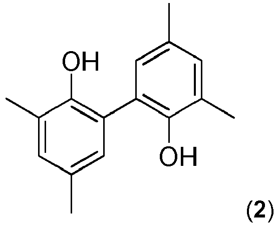 wobei die Lösung umfasst:
- ein Lösungsmittel,
- die Verbindung (**2**),
- Verunreinigungen, welche bei der Synthese angefallen sind;
b2) für den Fall, dass es sich bei dem Lösungsmittel von a2) nicht um Essigsäure handelt, Zugabe von Essigsäure;
c2) gießen der in b2) erhaltenen Lösung auf Eis oder zugeben von Eis zu der in b2) erhaltenen Lösung, so dass die Verbindung gemäß der Formel (**2**) als Feststoff ausfällt.

3. Verfahren nach einem der Ansprüche 1 oder 2,
umfassend den zusätzlichen Verfahrensschritt:
d) abfiltrieren des ausgefallenen Feststoffes.

4. Verfahren nach Anspruch 3
umfassend den zusätzlichen Verfahrensschritt:
e) lösen des Feststoffes aus d) in Ethylacetat und waschen der Ethylacetat-Lösung mit Wasser.

5. Verfahren nach Anspruch 4,
umfassend den zusätzlichen Verfahrensschritt:
f) trocknen der Lösung aus e).

6. Verfahren nach Anspruch 5,
umfassend den zusätzlichen Verfahrensschritt:
g) abdestillieren des Lösungsmittel aus der in f) erhaltenen Lösung.

7. Verfahren nach Anspruch 6,
umfassend den zusätzlichen Verfahrensschritt:
h) auflösen des in g) erhaltenen Rückstandes in Cyclohexan.

8. Verfahren nach Anspruch 7,
umfassend den zusätzlichen Verfahrensschritt:
i) abkühlen der Lösung aus h) auf eine Temperatur in dem Bereich von 15 °C bis 7 °C, so dass ein kristalliner Feststoff ausfällt.

9. Verfahren nach Anspruch 8,
umfassend den zusätzlichen Verfahrensschritt:
j) waschen des kristallinen Feststoffes aus i) mit Cyclohexan, wobei das Cyclohexan eine Temperatur aufweist in dem Bereich von 15 °C bis 7 °C.
